# EUROPÄISCHE PATENTSCHRIFT

(11) **EP 0 927 066 B1**
(45) Veröffentlichungstag und Bekanntmachung des Hinweises auf die Patenterteilung: **23.03.2005**
(21) Anmeldenummer: 98921262.6
(22) Anmeldetag: 18.05.1998
(51) Int. Cl.: A61N 2/02

(54) **VORRICHTUNG ZUR ERZEUGUNG VON MAGNETISCHEN WECHSELFELDERN ZUR INDUZIERUNG VON WIRBELSTRÖMEN IN EINEM ORGANISMUS**
DEVICE FOR GENERATING MAGNETIC ALTERNATING FIELDS TO INDUCE EDDY CURRENTS IN AN ORGANISM
DISPOSITIF POUR PRODUIRE DES CHAMPS ALTERNATIFS MAGNETIQUES AFIN D'INDUIRE DES COURANTS DE FOUCAULT DANS UN ORGANISME

(30) Priorität: 22.07.1997 AT 45897
(43) Veröffentlichungstag der Anmeldung: 07.07.1999
(73) Patentinhaber: Neuwirth, Gerald, 9800 Spittal (AT)
(72) Erfinder: Neuwirth, Gerald, 9800 Spittal (AT)
(74) Vertreter: Miksovsky, Alexander, Dipl.-Ing.
(86) Internationale Anmeldenummer: PCT/AT1998/000129
(87) Internationale Veröffentlichungsnummer: WO 1999/004854

(56) Entgegenhaltungen:
- EP-A- 0 084 019
- EP-A- 0 392 626
- EP-A- 0 459 401
- DE-A- 3 721 864
- US-A- 3 678 337

## Beschreibung

Die vorliegende Erfindung bezieht sich auf eine Vorrichtung zur Erzeugung von magnetischen Wechselfeldern zur Induzierung von Wirbelströmen in einem Organismus sowie auf eine Verwendung einer derartigen Vorrichtung, wobei die Vorrichtung zur Erzeugung und Abgabe von magnetischen Wechselfeldern einer einstellbaren, niedrigen Frequenz mit Impulsen mit steilen Flanken mit Oberwellenanteilen ausgebildet ist, wobei die magnetische Feldstärke der von der Vorrichtung abgegebenen, magnetischen Wechselfelder vorzugsweise einstellbar geringer als 300 A/m, insbesondere zwischen 100 und 250 A/m, gewählt ist.

Es sind unterschiedliche Vorrichtungen zur Erzeugung und Abgabe von elektromagnetischen Wechselfeldern zur Behandlung und Untersuchung von Organismen bekannt, wobei allgemein davon auszugehen ist, daß die Eindringtiefe derartiger elektromagnetischer Wechselfelder von der Frequenz abhängt. Eine Vorrichtung der eingangs genannten Art ist beispielsweise aus der EP-A 0 084 019 bekannt geworden. Allgemein kann das elektrische Feld hiebei vernachlässigt werden, solange sich die Frequenz in einem sehr niedrigen Bereich, d.h. im Bereich einer sogenannten extremely low frequency, befindet. Im Gegensatz dazu induziert ein magnetisches Wechselfeld im gesamten Organismus Wirbelströme, welche Ladungsverschiebungen in den Zellmembranen zur Folge haben, wodurch Reizungen des vegetativen Nervensystems hervorgerufen werden, die beispielsweise im Organismus vorhandene Blockierungen abbauen können.

Die vorliegende Erfindung zielt nun darauf ab, eine Vorrichtung zur Erzeugung von magnetischen Wechselfeldern zur Induzierung von Wirbelströmen in einem Organismus zur Verfügung zu stellen, mit welcher in konstruktiv einfacher Weise gezielt und gefahrlos in einem Organismus Wirbelströme erzeugt werden können, um durch die hiedurch hervorgerufenen Reizungen des vegetativen Nervensystems eine gezielte Beeinflussung von möglichen Ladungsverschiebungen in den Zellmembranen zu bewirken bzw. vorhandene Blockierungen abbauen bzw. mildern zu können.

Zur Lösung dieser Aufgaben ist die erfindungsgemäße vorrichtung im wesentlichen dadurch gekennzeichnet, daß die Vorrichtung in einem Kopfhörer aufgenommen ist. Eine erfindungsgemäße Vorrichtung läßt sich hiebei ohne weiteres in einem derartigen Kopfhörer bzw. einer allgemein im Bereich der Ohren anordenbaren Vorrichtung, gegebenenfalls mit einem entsprechend kleinbauenden Zusatzgerät, verwirklichen, so daß insgesamt eine leicht anwendbare und einsetzbare Konstruktion erzielbar ist. Während bei Behandlung lediglich eines Ohrs eine Ausbildung mit nur einer bipolaren Eisenkernspule denkbar ist, wenn beispielsweise nur in einem Ohr Ohrengeräusche auftreten, ist gemäß einer bevorzugten Ausführungsform vorgesehen, daß in jedem Lautsprecher des Kopfhörers jeweils eine bipolare Eisenkernspule enthalten ist.

Da magnetische Wechselfelder einer einstellbaren, niedrigen Frequenz von der erfindungsgemäßen Vorrichtung abgegeben werden, gelingt eine einfache Anpassung an die bei unterschiedlichen Organismen unterschiedliche Empfindlichkeit, wobei festgestellt wurde, daß die Empfindlichkeit bei jener Frequenz maximal ist, welche mit dem EEG-α-Rhythmus der zu behandelnden Person übereinstimmt. Durch die erfindungemäß gewählten, geringen Feldstärken ist weiters sichergestellt, daß bei unproblematischer und absolut gefahrloser Handhabung die gewünschten Effekte im Hinblick auf die Induzierung von Wirbelströmen im Organismus erzielbar sind. Dabei werden Impulse in Form von Wellen mit pulsierenden, steilen Flanken mit Oberwellenanteilen erzeugt und abgegeben, wobei durch diese induzierten und an den Organismus abgegebenen Impulse optimal im Organismus vorhandene Eigenimpulse angeregt und unterstützt werden, wenn die Frequenz der von außen aufgebrachten, magnetischen Wechselfelder mit diesen Eigenimpulsen synchron ist, wodurch die angestrebten Reizungen des vegetativen Nervensystems unterstützt werden.

Eine besonders einfache und konstruktiv leicht herstellbare Ausführungsform der erfindungsgemäßen Vorrichtung ist hiebei bevorzugt derart ausgebildet, daß die Vorrichtung einen astabilen Multivibrator umfassend zumindest einen Transistor und wenigstens eine bipolare Eisenkernspule zur Abgabe der Wechselfelder aufweist. Hiebei läßt sich mit an sich bekannten Elementen in relativ einfacher Form und kostengünstig herstellbar die Erzeugung und Abgabe der magnetischen Wechselfelder mit Impulsen mit steilen Wellenflanken mit einem Oberwellenanteil erzielen.

Eine erfindungsgemäße Vorrichtung läßt sich insbesondere zur Behandlung von Ohrengeräuschen, Schlafstörungen, Kopfschmerzen, Migräne, Hals- und Rückenwirbelsyndromen, Nervosität und Durchblutungsstörungen verwenden, wobei sich allgemein gezeigt hat, daß günstig wirkende Bereiche der magnetischen Wechselfelder mit einer einstellbaren, niedrigen Frequenz derart gewählt sind, daß die Frequenz unterhalb von 20 Hz, insbesondere zwischen 3 und 15 Hz, liegen, wie dies einer weiteren bevorzugten Ausführungsform der erfindungsgemäßen Vorrichtung entspricht. Allgemein wurde eine Unterteilung in im wesentlichen zwei behandlungsspezifische Bereiche gefunden, wobei ein Frequenzbereich zwischen 3 und 6 Hz allgemein beruhigend und krampflösend wirkt. Demgegenüber wirkt der Frequenzbereich zwischen 8 und 15 Hz im wesentlichen anregend, schmerzstillend und stabilisierend. Weiters kann davon ausgegangen werden, daß Frequenzen unter 8 Hz im wesentlichen Blutgefäßerweiterungen bewirken, während Frequenzen über 12 Hz allgemein zu Blutgefäßverengungen führen, wobei der oben angegebene, hohe Frequenzbereich nur angewandt werden sollte, wenn niedrigere Frequenzen wirkungslos sind.

Für eine weitere Verbesserung der Anpassung der einzustellenden Frequenz an die erwünschte Wirkung wird gemäß einer weiters bevorzugten Ausführungsform vorgeschlagen, daß ein insbesondere digitales Wobbeln der Frequenz um ± 25 %, vorzugsweise ± 10 %, vorgesehen ist.

Für eine baulich einfache Konstruktion und insbesondere zur Ausbildung von Vorrichtungen, welche unabhängig von einer Netzspannungsversorgung betrieben werden können, wird darüberhinaus gemäß ein weiters bevorzugten Ausführungsform vorgeschlagen, daß als Spannungsversorgung eine Niederspannungs-Gleichspannungsquelle, insbesondere eine 9-Volt-Gleichspannungsquelle, vorgesehen ist. Derart lassen sich auch in einfacher Weise mobile und tragbare Geräte unabhängig von einer Netzversorgung durch Vorsehen einer Batterie als Gleichspannungsversorgung ausbilden, was insbesondere bei der Anwendung zur Behandlung von Tinnitus von Vorteil ist.

Wie oben bereits angedeutet, liegt ein wesentliches Merkmal der erfindungsgemäßen Vorrichtung in der Verfügbarkeit von einstellbaren, niedrigen Frequenzen zur Behandlung unterschiedlicher Indikationen sowie zur Erzielung einer bestmöglichen Übereinstimmung der magnetischen Wechselfelder und den der zu behandelnden Person innewohnenden Eigenimpulse. In diesem Zusammenhang wird gemäß einer weiters bevorzugten Ausführungsform vorgeschlagen, daß die Vorrichtung eine Mehrzahl von Schaltern bzw. Auswahlmittel zur Einstellung einer gewünschten Frequenz aufweist. Derart läßt sich in einfacher Weise eine Anpassung an gewünschte Frequenzbereiche sowie die Erzielung einer möglichst genauen Übereinstimmung mit der Frequenz der Eigenimpulse der zu behandelnden Person erzielen.

Für eine weitere Vereinfachung bei der Handhabbarkeit und Überwachung der erfindungsgemäßen Vorrichtung ist weiters bevorzugt vorgesehen, daß die Vorrichtung wenigstens eine Anzeige, insbesondere eine LCD-Anzeige, aufweist. Zur weiteren Automatisierung des Einsatzes der erfindungsgemäßen Vorrichtung wird vorgeschlagen, daß die Vorrichtung einen Zeitgeber aufweist, wie dies einer weiteren bevorzugten Ausführungsform der Erfindung entspricht.

Die Erfindung wird nachfolgend anhand von in der beiliegenden Zeichnung schematisch dargestellten Ausführungsbeispielen näher erläutert. In dieser zeigen:
Fig. 1 ein schematisches Schaltungsdiagramm einer erfindungsgemäßen Vorrichtung;
Fig. 2 eine grafische Darstellung der mit der erfindungsgemäßen Vorrichtung erzeugten Impulse niedriger Frequenz mit pulsierenden, steilen Flanken mit Oberwellenanteilen;
Fig. 3 eine Abwandlung des Schaltungsdiagramms von Fig. 1 für den Einsatz in einem tragbaren Gerät; und
Fig. 4 eine weitere Schaltanordnung der erfindungsgemäßen Vorrichtung für einen Einsatz als Standgerät.

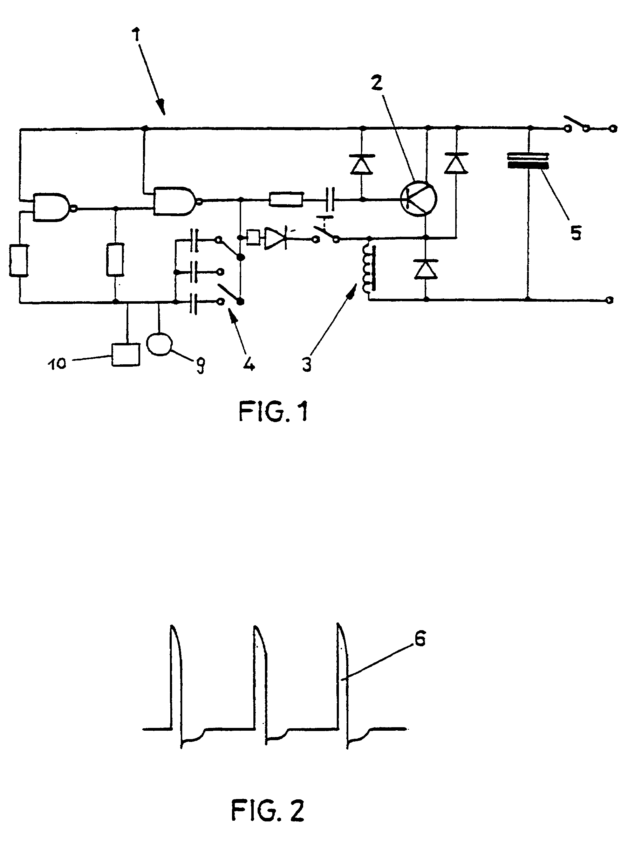

In Fig. 1 ist schematisch eine Schaltung eines astabilen Multivibrators 1 dargestellt, wobei ein Transistor mit 2 und eine bipolare Eisenkernspule allgemein mit 3 bezeichnet ist. Weiters ist eine Mehrzahl von Schaltern bzw. Auswahlelementen 4 und eine Gleichspannungsquelle 5 angedeutet. Mit dem in Fig. 1 dargestellten, astabilen Multivibrator werden steile, oberwellenartige Impulse 6 erzeugt, wie sie schematisch in Fig. 2 angedeutet sind. Die Wellen mit pulsierenden, steilen Flanken der Impulse 6 werden hiebei durch Oberwellenanteile bis in den Gigahertzbereich erzielt. Mit 9 ist zusätzlich wenigstens eine Anzeige angedeutet, während 10 einen integrierten bzw. koppelbaren Zeitgeber schematisch bezeichnet.

Zur Erzeugung der in Fig. 2 schematisch dargestellten, steilen, oberwellenartigen Impulse 6 leitet und sperrt der Transistor 2 im Rhythmus der ausgewählten Frequenz, wobei der impulsförmige Kollektorstrom des Transistors 2 den Kern der Eisenkernspule 3 entsprechend magnetisiert. Die magnetische Feldstärke kann hiebei unter 300 A/m und insbesondere zwischen 100 und 250 A/m gewählt sein.

Durch die im schematischen Schaltbild der Fig. 1 angedeuteten, drei Schalter 4 lassen sich durch Kombination der Schalter die nachfolgend angeführten Frequenzen erzielen, wobei in der nachfolgenden Tabelle in der linken Spalte jeweils die zur Erzielung der in der rechten Spalte angegebenen Frequenzen zu betätigenden Schalter angeführt sind.

| Schalter | Hz |
|---|---|
| 1 | 4,5 |
| 2 | 9,5 |
| 3 | 15 |
| 1 und 2 | 3 |
| 1 und 3 | 3,5 |
| 2 und 3 | 6 |
| 1 und 2 und 3 | 25 |

Alternativ zu einer Schalteranordnung, wie sie in Fig. 1 dargestellt ist, mit drei Schaltern bzw. Auswahlelementen kann bei einer einfachen Ausführungsform bei Vorsehen von nur einem Schalter beispielsweise dadurch eine Frequenz von 5 bzw. 10 Hz einstellbar sein.

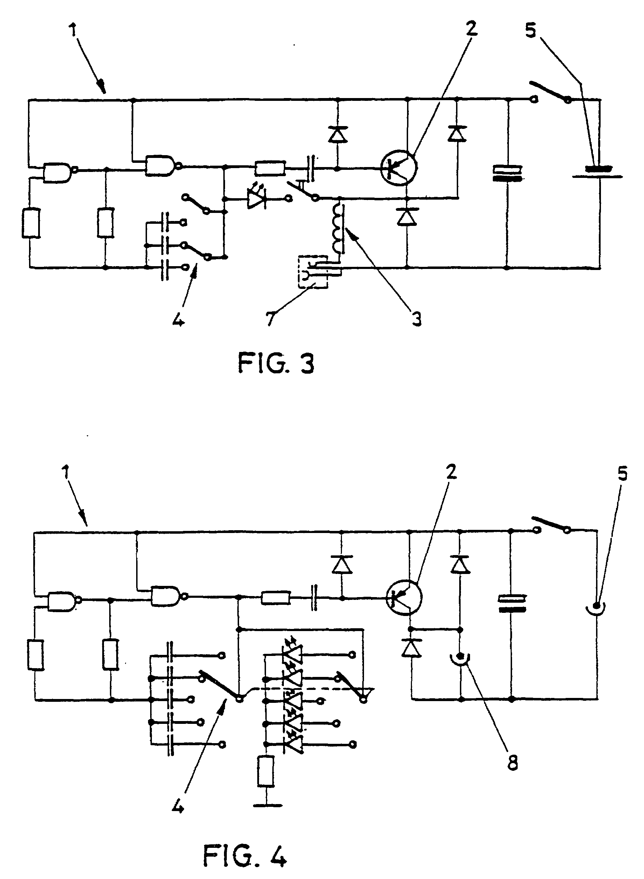

Fig. 3 ist eine Abwandlung bzw. Weiterbildung der schematischen Schaltung des astabilen Multivibrators 1 von Fig. 1, wobei die Bezugszeichen von Fig. 1 für gleiche Elemente beibehalten wurden. Die schematische Schaltung gemäß Fig. 3 ist hiebei insbesondere für ein tragbares zum Einsatz bei der Behandlung von Tinnitus gedacht. Die Anordnung der Schalt- bzw. Auswahlelemente 4 ist hiebei so gewählt, daß zwischen vier verschiedenen Frequenzen gewählt werden kann. Als Gleichspannungsquelle 5 kommt bei der Schaltung gemäß der Fig. 3 eine Batterie zum Einsatz. Mit dem in Fig. 3 dargestellten astabilen Multivibrator werden ebenso wie in Fig. 1 steile, oberwellenartige Impulse 6 erzeugt, wie sie schematisch in Fig. 2 angedeutet sind, wobei für die Übertragung dieser Impulse auf einen Kopfhörer in Fig. 3 eine Buchse 7 für den Anschluß eines externen Kopfhörers angedeutet ist. Wie oben bereits angeführt, kann insbesondere zur Behandlung von Ohrengeräuschen eine derartige Vorrichtung in einem Kopfhörer integriert sein, wobei zu diesem Zweck in jedem Kopfhörer jeweils eine bipolare Eisenkernspule 3 vorgesehen ist.

Die schematische Schaltungsanordnung gemäß Fig. 4, welche eine Weiterbildung der Schaltungsanordnung in Fig. 1 und 3 darstellt, ist derart ausgeführt, daß fünf Schalter bzw. Auswahlelemente 4 zum Einsatz gelangen. Durch die im schematischen Schaltbild der Fig. 4 angedeuteten fünf Schalter 4 lassen sich die nachfolgend angeführten Frequenzen erzielen, wobei in der nachfolgenden Tabelle in der linken Spalte jeweils die zur Erzielung der in der rechten Spalte angegebenen Frequenzen zu betätigenden Schalter angeführt sind.

| Schalter | Hz |
|---|---|
| 1 | 3 |
| 2 | 4,5 |
| 3 | 6 |
| 4 | 9,5 |
| 5 | 15 |

In der Schaltungsanorndung gemäß Fig. 4 ist keine bipolare Eisenkernspule 3 angedeutet, da diese in extern zu tragenden, nicht dargestellten Kopfhörern angeordnet sind. Die Kopfhörer werden hiebei über die Buchse 8 angesteckt und die Eisenkernspulen auf die oben angegebene Weise angeregt.

In vorteilhafter Weise kann die Schaltungsanordnung gemäß Fig. 4 in ein Standgerät eingebaut werden, sodaß die schematisch dargestellte Gleichspannungsquelle 5 als Buchse für ein Netzgerät mit beispielsweise 9 V ausgebildet sein kann, um das Gerät für einen Langzeiteinsatz zu adaptieren.

Neben der Ausbildung in Form eines Kopfhörers kann alternativ vorgesehen sein, eine derartige Vorrichtung zur Erzeugung von magnetischen Wechselfeldern zur Induzierung von Wirbelströmen in einem Organismus als mobiles Gerät unmittelbar am Körper, beispielsweise in einer Jackentasche oder einem am Körper zu tragenden Beutel, zu tragen. Alternativ kann vorgesehen sein, die Vorrichtung in ein Kopfkissen oder ein ähnliches Element zu integrieren, welches, beispielsweise während dem Liegen, im Bereich des Halses, insbesondere zur Behandlung von Kopfschmerzen oder Hals- und Rückwirbelsyndromen, angeordnet wird.

## Patentansprüche

1. Vorrichtung (1) zur Erzeugung von magnetischen Wechselfeldern zur Induzierung von Wirbelströmen in einem Organismus, wobei die Vorrichtung (1) zur Erzeugung und Abgabe von magnetischen Wechselfeldern einer einstellbaren, niedrigen Frequenz mit Impulsen (6) mit steilen Flanken mit Oberwellenanteilen ausgebildet ist, wobei die magnetische Feldstärke der von der Vorrichtung (1) abgegebenen, magnetischen Wechselfelder vorzugsweise einstellbar geringer als 300 A/m, insbesondere zwischen 100 und 250 A/m, gewählt ist, **dadurch gekennzeichnet, daß** die Vorrichtung in einem Kopfhörer aufgenommen ist.

2. Vorrichtung nach einem der Ansprüche 1, **dadurch gekennzeichnet, daß** in jedem Lautsprecher des Kopfhörers jeweils eine bipolare Eisenkernspule (3) enthalten ist.

3. Vorrichtung nach Anspruch 1 oder 2, **dadurch gekennzeichnet, daß** die Vorrichtung einen astabilen Multivibrator (1) umfassend zumindest einen Transistor (2) und wenigstens eine bipolare Eisenkernspule (3) zur Abgabe der Wechselfelder aufweist.

4. Vorrichtung nach Anspruch 1, 2 oder 3, **dadurch gekennzeichnet, daß** die Frequenz unterhalb von 20 Hz, insbesondere zwischen 3 und 15 Hz, gewählt ist.

5. Vorrichtung nach einem der Ansprüche 1 bis 4, **dadurch gekennzeichnet, daß** ein insbesondere digitales Wobbeln der Frequenz um ± 25 %, vorzugsweise ± 10 %, vorgesehen ist.

6. Vorrichtung nach einem der Ansprüche 1 bis 5, **dadurch gekennzeichnet, daß** als Spannungsversorgung (5) eine Niederspannungs-Gleichspannungsquelle, insbesondere eine 9-Volt-Gleichspannungsquelle, vorgesehen ist.

7. Vorrichtung nach einem der Ansprüche 1 bis 6, **dadurch gekennzeichnet, daß** die Vorrichtung eine Mehrzahl von Schaltern (4) bzw. Auswahlmittel zur Einstellung einer gewünschten Frequenz aufweist.

8. Vorrichtung nach einem der Ansprüche 1 bis 7, **dadurch gekennzeichnet, daß** die Vorrichtung wenigstens eine Anzeige (9), insbesondere eine LCD-Anzeige, aufweist.

9. Vorrichtung nach einem der Ansprüche 1 bis 8, **dadurch gekennzeichnet, daß** die Vorrichtung einen Zeitgeber (10) aufweist.

10. Verwendung der Vorrichtung nach einem der Ansprüche 1 bis 9 zur Behandlung von Ohrengeräuschen, Schlafstörungen, Kopfschmerzen, Migräne, Hals- und Rückenwirbelsyndromen, Nervosität und Durchblutungsstörungen.

## Claims

1. An apparatus (1) for producing alternating magnetic fields for inducing eddy currents in an organism, wherein the apparatus is designed to produce and deliver alternating magnetic fields of an adjustable, low frequency at pulses (6) having steep pulse edges with harmonic wave portions, wherein the magnetic field strength of the alternating magnetic fields delivered by the apparatus (1) is chosen to be smaller than 300 A/m and, in particular, between 100 and 250 A/m, preferably in an adjustable manner, **characterized in that** the apparatus is integrated in a headphone.

2. An apparatus according to claim 1, **characterized in that** in each speaker of the headphone one bipolar iron core coil (3) each is contained.

3. An apparatus according to claim 1 or 2, **characterized in that** the apparatus comprises an astable multivibrator (1) including at least one transistor (2) and at least one bipolar iron core coil (3) for delivering the alternating fields.

4. An apparatus according to claim 1, 2 or 3, **characterized in that** the frequency is chosen below 20 Hz and, in particular, between 3 and 15 Hz.

5. An apparatus according to any of the claims 1 to 4, **characterized in that** in particular digital wobbling of the frequency by ± 25 % and, preferably, ± 10 % is provided.

6. An apparatus according to any one of claims 1 to 5, **characterized in that** a low voltage d.c. voltage source (5), in particular a 9 volt d.c. voltage source, is provided as a power supply (5).

7. An apparatus according to any one of claims 1 to 6, **characterized in that** the apparatus comprises a plurality of switches (4) or selection means for adjusting a desired frequency.

8. An apparatus according to any one of claims 1 to 7, **characterized in that** the apparatus comprises at least one display (9) and, in particular, an LCD display.

9. An apparatus according to any one of claims 1 to 8, **characterized in that** the apparatus comprises a timer (10).

10. The use of the apparatus according to any one of claims 1 to 9 for treating ear noises or tinnitus, sleep disturbances, headaches, migraine, cervical vertebral and spinal vertebral syndromes, nervousness and circulatory disturbances.

## Revendications

1. Dispositif (1) pour produire des champs alternatifs magnétiques afin d'induire des courants de Foucault dans un organisme, le dispositif (1) étant configuré pour produire et émettre des champs alternatifs magnétiques d'une basse fréquence réglable avec des impulsions (6) à fronts raides renfermant des fractions d'harmoniques, l'intensité des champs alternatifs magnétiques émis par le dispositif (1) étant de préférence choisie réglable au-dessous de 300 A/m, en particulier entre 100 et 250 A/m, **caractérisé en ce que** le dispositif est logé dans un casque d'écoute.

2. Dispositif suivant l'une des revendications 1, **caractérisé en ce que** chaque haut-parleur du casque d'écoute comporte une bobine à noyau de fer bipolaire (3).

3. Dispositif suivant l'une des revendications 1 et 2, **caractérisé en ce que** le dispositif présente un multi-vibrateur astable (1) comprenant au moins un transistor (2) et au moins une bobine à noyau de fer bipolaire (3) pour émettre les champs alternatifs.

4. Dispositif suivant l'une des revendications 1, 2 et 3, **caractérisé en ce que** la fréquence est choisie au-dessous de 20 Hz, en particulier entre 3 et 15 Hz.

5. Dispositif suivant l'une des revendications 1 à 4, **caractérisé en ce qu'**il est prévu une vobulation en particulier numérique de la fréquence de ± 25 %, de préférence ± 10 %.

6. Dispositif suivant l'une des revendications 1 à 5, **caractérisé en ce qu'**il est prévu comme alimentation en tension (5) une source de tension continue à basse tension, en particulier une source de tension continue de 9 V.

7. Dispositif suivant l'une des revendications 1 à 6, **caractérisé en ce que** le dispositif présente une pluralité d'interrupteurs (4) ou de moyens de sélection pour le réglage d'une fréquence souhaitée.

8. Dispositif suivant l'une des revendications 1 à 7, **caractérisé en ce que** le dispositif présente au moins un affichage (9), en particulier un affichage à cristaux liquides.

9. Dispositif suivant l'une des revendications 1 à 8, **caractérisé en ce que** le dispositif présente une horloge (10).

10. Utilisation du dispositif suivant l'une des revendications 1 à 9 pour le traitement de bruits d'oreilles, d'insomnies, de maux de tête, de migraines, de syndromes des vertèbres cervicales et dorsales, de nervosité et de mauvaise circulation sanguine.
